# EUROPEAN PATENT APPLICATION

(11) **EP 0 766 952 A2**
(43) Date of publication of application: **09.04.1997**
(21) Application number: 96307278.0
(22) Date of filing: 04.10.1996
(51) Int. Cl.: A61F 2/16

(54) **Intraocular lenses**

(30) Priority: 06.10.1995 GB 9520457
(71) Applicant: Rayner Intraocular Lenses Limited, Amersham, Buckinghamshire (GB); IH Laboratories Ltd., Gravesend, Kent, DA13 OLT (GB)
(72) Inventor: Highgate, Donald James, The Wilderness, Dorking, Surrey, RH5 6NS (GB); Frankland, John David, IH Laboratories Ltd., Meopham, Gravesend, Kent, DA13 0LT (GB); Parker, John Henry, IH Laboratories Ltd., Meopham, Gravesend, Kent, DA13 0LT (GB); Collins, Ian, Rayner Intraocular Lenses Ltd., Amersham, Buckinghamshire (GB)
(74) Representative: Perry, Robert Edward

(57) **Abstract**

An intraocular lens of the type comprising an optic and one or more attached haptics is characterised in that the attachment of the or each haptic to the optic lies in the median plane of the lens/optic when folded or unfolded.

Alternatively or in addition, at least the centre of the optic exhibits slower shape-recovery.

## Description

### Field of the Invention

This invention relates to intraocular lenses.

### Background of the Invention

An intraocular lens typically comprises an optic and two or more flexible haptics that act as aids to centration. The use of such a lens presents two conflicting requirements, i.e. that the lens should be introduced through a small incision, to minimise damage to the eye, but be sufficiently large to be stabilised in the eye after insertion.

One solution to this problem has been to use foldable lenses. These are usually delivered hydrated, it being left to the surgeon to fold them at the point of use. The suspension system (haptic) gradually adopts the necessary configuration, after insertion, in order that the optic should occupy the correct position. There appears to be a consensus that the lens should not unfold too rapidly, but beyond this there appears to be the presumption that the whole lens should recover from its folded or pre-stressed form in a few minutes.

As indicated above, the design of haptics has been driven by the need to insert such structures through a small incision (and possibly to remove it later, e.g. if a problem arises during the operation). Practically, this has militated against anything other than two-limb systems which can be inserted along one line. This is because haptics are generally of semi-rigid or springy materials.

US-A-4159546 discloses intraocular lenses of this type, e.g. comprising an optic of polymethyl methacrylate (PMMA) and haptics of polypropylene. A three-haptic lens is also illustrated, and it is indicated that "a three-point fixation will be achieved once the lens is implanted, which may provide even more sufficient centering and fixation". No solution is provided to the admitted problem that the "implantation procedure may be more difficult because of the additional strand".

EP-B-0336318 discloses intraocular lenses comprising shape-memory polymers. The glass transition temperature of the polymer is 40-60°C, i.e. above body temperature. In practice, therefore, such a lens is unlikely to be of much value.

EP-B-0308130 describes thermolabile lenses composed of copolymers of methacrylate and acrylate esters which are respectively relatively hard and relatively soft at body temperature, cross-linked with diacrylate ester. The difference between insertion and eye temperatures is intended to provide the necessary unfolding after insertion, owing to the thermolability of the polymer.

EP-B-0269288 also discloses thermolabile intraocular lenses. The softening temperature is between 0 and 42°C. The lenses are prepared for insertion by heating to reduce one dimension and then cooling the lens to a temperature at least 5°C less than the softening temperature.

WO-A-9407686 describes hydrogels prepared from a variety of particular vinyl monomers. The thermolability of the hydrogels renders them suitable for the construction of intraocular lenses that expand after insertion.

### Summary of the Invention

It has now been appreciated that the requirements for intraocular lenses can be met, at least in part, by distinguishing between the time necessary for the support systems to take effect, and the time necessary for the core to achieve optical shape. Thus the haptics may gain the desired shape within 30 to 120 seconds, e.g. 1 to 2 minutes, while the optic may take 6 to 12 hours to gain its shape. Thus the rate of recovery of the haptic may be a factor of 180 or more faster than that of the optic.

Further, the invention utilises the availability of high strength, high notch tear strength materials which are hydrophilic or thermolabile, to remove the existing restrictions on two-limb systems. The necessary physical parameters for the suspension systems are effectively defined in terms of the force:diameter relationship.

An intraocular lens of the type to which this invention relates, comprises an optic and one or more haptics (usually at least two), wherein at least the one or more haptics and optionally also the periphery of the optic comprise a shape-recovery material such that the lens achieves centration in use. In one aspect of the invention, at least the centre of the optic is relatively rigid, and/or exhibits different shape-recovery. In a second or additional aspect of the invention, the one or more haptics are attached to the optic at a plurality of points that lie in the median plane of the lens/optic. This remains the case, whether the lens is folded, ready for insertion, or unfolded, e.g. in use, and during unfolding or relaxation.

By means of the invention, it is now possible to use a blank of specific properties to fabricate lenses having a conventional material as the optic, surrounded by and supported by a different, high strength material from which the support system (haptics) can be fabricated. One benefit of this system is the provision of a lens made principally (by weight fraction) from a well known material, while providing high strength high notch tear strength properties for the edge of the lens body and the haptics. Another benefit is the provision of an automatic "angulation" for the lens, by deliberately designing the core to have a higher expansion ratio than the edge. A further benefit is that, by using prestressed high tear strength materials, simple or complex multi-haptic designs are feasible.

### Brief Description of the Drawings

In the accompanying drawings (which are given by of example only):
Figs. 1A, 1C and 1E are schematic, plan views of an intraocular lens that embodies the present invention, respectively in a folded state and then after increasing periods of shape recovery in use;
Figs. 1B, 1D and 1F are respectively sections along X-X in Fig. 1A, Y-Y in Fig. 1C and Z-Z in Fig. 1E;
Fig. 2 is a plan view of a second intraocular lens that embodies the invention;
Fig. 3 is a graph representing the force needed to compress the lens of Fig. 2;
Fig. 4 is a partial view of a blank from which a third lens embodying the invention may be made;
Fig. 5 is a schematic side view of the third embodiment, after expansion, in use; and
Figs. 6A and 6B, and 7A and 7B, are each associated plan and side views of respective further lenses embodying this invention.

### Description of the Invention

In accordance with one principal aspect of the invention, the outer part of the lens, i.e. the haptics and optionally also the periphery of the optic, is formed of material which is constrained before insertion of the lens, and recovers its desired shape after insertion into the eye. The materials can be chosen so that the haptics (which have a relatively very small volume compared to the optic) can recover very quickly, giving centration of the lens, in use, within 2 minutes. This is independent of the nature of the inner part of the lens, i.e. the whole optic or at least its centre. The inner part of the lens can thus be formed of a material that is conventional in the field, and which may be familiar to the surgeon. Such materials include methacrylate polymers such as the 'soft' polymer of 2-hydroxyethyl methacrylate (HEMA), the relatively rigid PMMA, and also MMA-vinylpyrolidone.

The centre of the lens may itself be formed of a shape-recovery material, but the rate of recovery does not affect the ability to achieve relatively fast recovery of the outer part of the lens, and thus relatively rapid centration, in use. Alternatively, the inner part of the lens may not be such a material, e.g. relatively rigid, but since the outer part of the lens can be constrained, the inner part only determines the essential size of the lens on insertion. Thus the desire for a relatively small incision in the eye can be met.

The haptics may be formed of a shape-recovery material that is, for example, thermolabile and/or hydrophilic. Various thermolabile materials are known, including those in the prior art described above. Suitable hydrophilic materials include acrylonitrile-vinylpyrrolidone (AN-VP), as described in GB-A-1566552. Also as described more fully in that specification, such materials can be pre-stressed, so that they recover a shape, on hydration, that is very accurately controllable. Thus a lens of the invention can be produced from materials such that, while it is dry, it does not need constraint in order to adopt a conformation suitable for insertion. After insertion, it regains the desired shape, on hydration.

As indicated above, shape-recovery may be caused by hydration, i.e. the liquid in the eye, or as a consequence of the eye's temperature being higher than ambient temperature. In order to accentuate the temperature difference, and thus allow the use of a wide range of materials, it may be desirable to cool a thermolabile material before insertion. That constraint is avoided by the use of hydrophilic materials, but it is a particular advantage of the use of, say, AN-VP that shape-recovery is enhanced by its inherent thermolability.

In an alternative means of providing shape-recovery, the lens may be formed of a conventional material such as polymethyl methacrylate, e.g. as described in US-A-4159546, but in which the outer part of the lens is coated or otherwise treated with a material that constrains its shape before and during insertion, and is dissolved or otherwise functionalised on insertion, so that the outer part of the lens can regain the desired shape, in use, within a period of time, e.g. up to 2 minutes, that can be controlled by the nature of the modification. For example, suitable modification includes a polyethylene oxide delivery sheath that dissolves in the eye. Alternatively, a hydrophilic material may be applied as a coating or interpenetrant that is applied and prestressed (thus holding the haptic in a new, stressed state), and which softens as it hydrates, allowing the haptic to relax controllably, to assume its unstressed state.

It is preferable that the degree of recovery, from size on insertion to size in use, should be such that the axial extension/lateral reduction is at least 2:1, more preferably at least 3:1, and most preferably at least 4:1, e.g. about 5:1. Such ratios can be achieved using, for example, AN-VP.

For ease of manufacture, the lens is preferably formed from a single blank. After forming, e.g. if a rigid material is used, it can then be coated or sheathed. Alternatively, if the lens is formed from two materials, e.g. AN-VP in the outer part and HEMA on the inner part, the two materials are preferably sufficiently well matched that high tensile strength is retained. Preferably, the two materials interpenetrate. It is particularly desirable that the materials, at least of the haptic and optic periphery, should have high notch tear strength.

Preferred characteristics of materials used in this invention are a tensile strength of at least 30, e.g. 40 to 50, kg/cm²; a tear strength of at least 20, e.g. 30 to 40, kg/cm²; a notch tear strength at least 70% of the smooth surface tensile strength; and an elongation at break of at least 200, more preferably at least 300, e.g. 400 to 500,%. These criteria are satisfied by the materials exemplified herein.

A second principal aspect of the present invention is the provision of one or more haptics attached to the optic at a plurality of points that lie in the median plane of the lens/optic. Although this has previously been illustrated, for rigid materials, in US-A-4159546, there is no practical means of inserting such lenses through small incisions, if at all. However, the present invention allows folding, taking advantage of the very low relative volume of the haptics. By folding the lens such that the haptics lie substantially parallel to or substantially in the axis of insertion, the lens can be introduced through a small insertion, without the haptics immediately springing out after insertion. On the contrary, the use of appropriate materials allows the haptics to assume their desired shape, in the eye, in a controlled manner. Accordingly, lenses of the invention define an allowable class of folding.

Further, lenses of the invention can practically utilise three haptics, thereby ensuring optimum centration. As an alternative, one or two haptics may be used, and which provide satisfactory centration because they need not be especially thin; they may be looped or flap-like (as shown in the accompanying drawings). More generally, each haptic preferably defines an aperture, whereby the or each haptic may be compressed in the median plane of the lens. For example, there may be two haptics attached to the optic along opposed arcs thereof.

There are essentially two ways by which lenses according to the present invention may be made, ready for use. In one method, the "apparent" cross-section of the lens is reduced by increasing the axial length. This is readily achievable by the use of materials such as AN-VP. Alternatively, the ability to pre-stress may enable any desired shape to be made and polished, by pre-stressing it into a simple form, for cutting and polishing. More generally, lenses of the invention may be made by moulding or casting. In one method, a dry lens may be produced, by polishing, lathing, casting, or any combination of such methods, hydrated, deformed and dried. Hydraulic stressing allows appropriate fixation. In another method, the lens may be deformed, constrained in a frame, and delivered in a hydrated, soft form. A pre-stressed hydrophilic material may advantageously be subjected to a short drying step immediately prior to insertion, e.g. under an air jet for 20 seconds. The relatively dry surface thus provides for hydration substantially only at the surface, on insertion.

Embodiments of the invention will now be described by way of example only, with reference to the accompanying drawings.

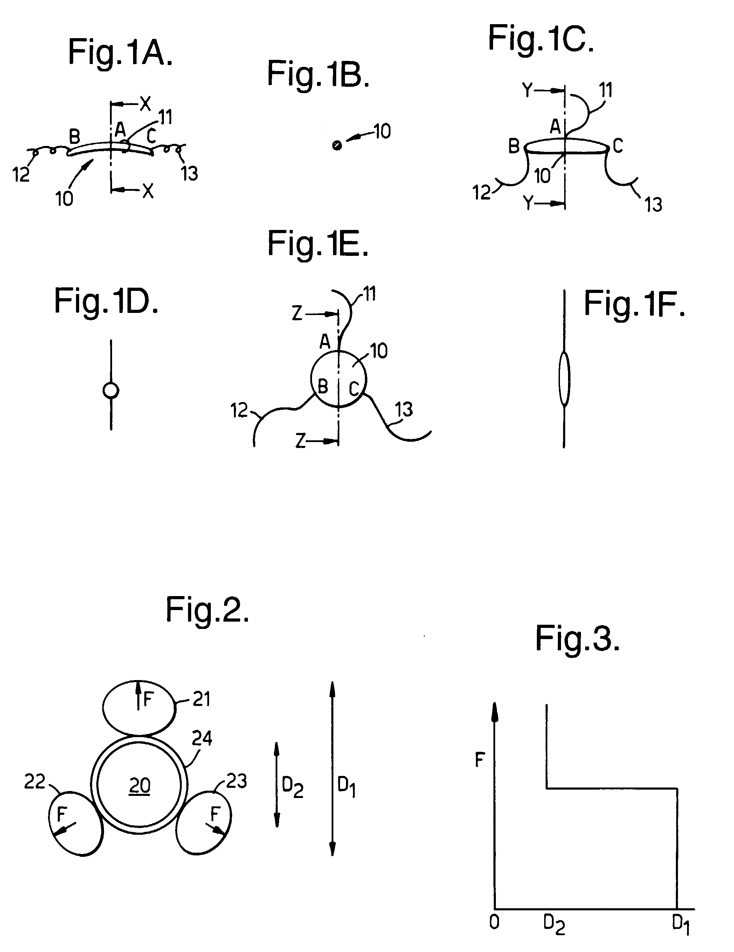

Fig. 1A shows a lens having an optic 10 and haptics 11, 12 and 13, respectively attached at points A, B and C, in a folded configuration that has a small, substantially circular cross-section (see Fig. 1B), ready for delivery. Fig. 1C shows the same lens, 1-2 minutes after insertion: the haptics have recovered their desired shape while the optic is substantially unchanged. As shown in Fig. 1D, the relatively bulky optic attains its desired shape, but after a much longer time, e.g. 6-12 hours.

Fig. 2 shows an embodiment of the invention having larger haptics than have previously been possible. The whole lens has a diameter D₁, while the optic 20 has a diameter D₂. There are three loop-like haptics 21, 22, 23 attached to the relatively high-strength periphery 24 of the optic. Fig. 3 shows the force F required to attain the given diameter.

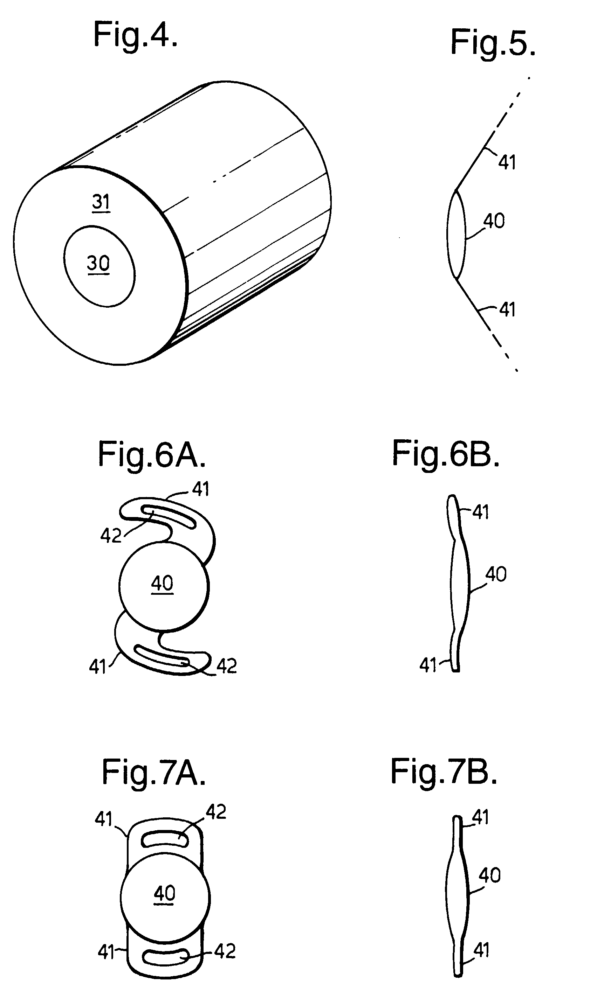

Fig. 4 shows a two-material blank comprising a core 30 and a sheath 31, e.g. of HEMA and AN-VP, respectively. Between the materials, a sharp interface is shown (D<D_{optic}). Alternatively, there may be a graded interface, if control over refractive index is required, e.g. for bifocal or vari-focal lenses. If the expansion ratio of the core is greater than the expansion ratio of the periphery, then the haptics are automatically set at an angle that can be controlled, as desired; see Fig. 5.

Fig. 5, Figs. 6A and 6B, and Figs. 7A and 7B, show respective intraocular lenses each comprising a core 40 and haptics 41. The haptics of the lenses shown in Figs. 6 and 7 each define an aperture 42. The two haptics 41 in each case are attached along opposed arcs of the optic 40. The haptics can be compressed in the median plane of the lens, substantially without buckling, to a suitable diameter for insertion, under a realistic compression force of 2-4 mN.

## Claims

1. An intraocular lens of the type comprising an optic and one or more attached haptics, and which comprises a shape-recovery material such that the lens achieves centration in use, wherein the attachment of the or each haptic to the optic lies in the median plane of the lens/optic in the folded or unfolded state.

2. An intraocular lens of the type comprising an optic and one or more attached haptics, wherein the one or more haptics and optionally also the periphery of the optic comprise a shape-recovery material such that the lens achieves its centration in use, and at least the centre of the optic exhibits slower shape-recovery.

3. An intraocular lens which has the characteristics of both claims 1 and 2.

4. A lens according to any preceding claim, wherein the shape-recovery material is a stressed hydrophilic material.

5. A lens according to any preceding claim, wherein the shape-recovery material is thermolabile.

6. A lens according to any preceding claim, wherein the shape-recovery material undergoes, on recovery, an axial extension/lateral reduction with a contraction ratio of at least 4:1.

7. A lens according to any preceding claim, wherein the shape-recovery material is acrylonitrile-vinylpyrrolidone.

8. A lens according to any preceding claim, wherein the or each haptic is coated such that it exhibits shape-recovery.

9. A lens according to claims 1 to 7, wherein at least the or each haptic is formed of the shape-recovery material, and at least the centre of the optic is of a material that exhibits slower shape-recovery.

10. A lens according to any preceding claim, wherein the material of at least the optic is a methacrylate polymer.

11. A lens according to any preceding claim, which comprises more than one haptic, and which is constrained in a configuration such that the optic is folded and the haptics lie substantially parallel.

12. A lens according to any preceding claim, wherein the or each haptic defines an aperture, whereby the or each haptic may be compressed in the median plane of the lens.

13. A lens according to any preceding claim, which comprises two haptics attached to the optic along opposed arcs thereof.
